# EUROPÄISCHE PATENTSCHRIFT

(11) **EP 3 209 155 B1**
(45) Veröffentlichungstag und Bekanntmachung des Hinweises auf die Patenterteilung: **15.08.2018**
(21) Anmeldenummer: 15793684.0
(22) Anmeldetag: 20.10.2015
(51) Int. Cl.: A44C 5/00, A41D 20/00, A61F 7/00, A61F 7/02

(54) **TRAGBARE VORRICHTUNG ZUR KÜHLUNG**
WEARABLE APPARATUS FOR COOLING
DISPOSITIF DE REFROIDISSEMENT PORTATIF

(30) Priorität: 20.10.2014 AT 507502014
(43) Veröffentlichungstag der Anmeldung: 30.08.2017
(73) Patentinhaber: Chillea OG, 1020 Wien (AT)
(72) Erfinder: GEYER, Michael, A-1120 Wien (AT); WALLERBERGER, Mark, A-1030 Wien (AT)
(74) Vertreter: KLIMENT & HENHAPEL
(86) Internationale Anmeldenummer: PCT/AT2015/050262
(87) Internationale Veröffentlichungsnummer: WO 2016/061603

(56) Entgegenhaltungen:
- EP-A2- 0 050 473
- WO-A1-97/13481

## Beschreibung

Die Erfindung betrifft eine tragbare Kühlvorrichtung mit einem Armband und einem am Armband angeordneten Kühler gemäß dem Oberbegriff von Anspruch 1.
Bei warmen Umgebungstemperaturen ist eine Kühlung des Körpers erwünscht. Falls eine Kühlung der Umgebungsluft selbst nicht gegeben ist, kann eine Kühlung der Hautoberfläche vorgenommen werden. Hierzu sind Hilfsmittel wie Fächer oder dergleichen bekannt, die durch einen Luftstrom auf die Hautoberfläche einen Kühlungseffekt erzielen. Des Weiteren ist eine tragbare Kühlvorrichtung mit einem Armband und einem am Armband angeordneten Kühler bekannt, wobei der Kühler als Peltier-Element ausgeführt ist, z.B. im Dokument DE 696 18 972. Peltier-Elemente zeichnen sich jedoch durch einen hohen Energiebedarf aus, sodass tragbare Geräte mit Peltier-Elementen nur kurze Betriebszeiten und vergleichsweise lange Ladezeiten aufweisen. Zudem muss das Peltier-Element in dichter Anlage zur Hautoberfläche verwendet werden, was mitunter als unangenehm empfunden wird.
Es ist daher das Ziel der Erfindung eine tragbare Kühlvorrichtung mit einem Armband und einem am Armband angeordneten Kühler bereitzustellen, die einen kleineren Stromverbrauch als bekannte Geräte aufweist, und somit bei gleichen Ladezeiten längere Betriebszeiten. Zudem soll die erfindungsgemäße Kühlvorrichtung unauffällig zu tragen sein und auch etwa im beruflichen Bereich bei entsprechender Bekleidung und üblicher Körperhaltung, etwa bei Arbeiten an einem Computer einem auf der Tischplatte aufliegenden Handgelenk, verwendbar sein.
Diese Ziele werden durch die Merkmale von Anspruch 1 verwirklicht. Anspruch 1 sieht bei einer tragbaren Kühlvorrichtung mit einem Armband und einem am Armband angeordneten Kühler erfindungsgemäß vor, dass der Kühler als Luftstromerzeuger für einen in eine Ausströmrichtung des Kühlers gerichteten Luftstrom ausgeführt ist, wobei der Luftstromerzeuger in einer am Armband befestigten oder angeformten Halterung angeordnet ist, die zumindest einen in Ausströmrichtung abstehenden Fortsatz aufweist, der zumindest abschnittsweise im oder am Luftstrom oder den Luftstrom zumindest abschnittsweise umschließend verläuft.
Erfindungsgemäß ist somit ein in einer Halterung angeordneter Luftstromerzeuger vorgesehen, wobei die Ausströmrichtung des Luftstromes bei geschlossenem Armband nach innen orientiert ist, also bei angelegtem Armband in Richtung der Hautoberfläche. Entscheidend für die Funktionsfähigkeit der erfindungsgemäßen Kühlvorrichtung ist dabei die Aufrechterhaltung eines geeigneten Luftstromes in Ausströmrichtung, der erfindungsgemäß mithilfe des zumindest einen in Ausströmrichtung abstehenden Fortsatzes der Halterung sichergestellt wird. Dieser Fortsatz verläuft zumindest abschnittsweise im oder am Luftstrom oder umschließt zumindest abschnittsweise den Luftstrom. Auf diese Weise wird bei angelegtem Armband wie noch näher ausgeführt werden wird ein Kühl- oder Abgabebereich für den Luftstrom geschaffen, wobei durch das Anströmen der Hautoberfläche mit dem kühlenden Luftstrom ein maximaler Kühleffekt erreicht wird. Das Armband wird in üblicher Weise im Handgelenksbereich angelegt, wobei sich der Luftstromerzeuger über dem Pulspunkt des Handgelenks befindet, also jenem Bereich, in dem der Puls gefühlt werden kann. Die Kühlung von Pulspunkten hat einen großen Einfluss auf das subjektive Wärmeempfinden des Menschen. Durch die angeströmte Luft fühlt sich der Träger des erfindungsgemäßen Armbandes subjektiv kühler, ohne dass der gesamte Körper gekühlt werden muss. Einer der empfindlichsten Pulspunkte liegt dabei an der Handwurzel, was durch die erfindungsgemäße Kühlvorrichtung ausgenutzt wird.

Die Halterung des Luftstromerzeugers kann auf ihrer bei geschlossenem Armband nach außen orientierten Seite aber auch mit einer Abdeckung versehen sein, die luftdurchlässige Bereiche aufweist, durch die Luft in die Kühlvorrichtung angesaugt werden kann. Diese luftdurchlässigen Bereiche können entweder durch Perforationen in einer an sich luftundurchlässigen Abdeckplatte, oder durch eine Luftdurchlässigkeit des verwendeten Materials der Abdeckung erreicht werden. Die Halterung des Luftstromerzeugers kann auf ihrer bei geschlossenem Armband nach außen orientierten Seite auch mit einer Abdeckung versehen sein, die mit der Halterung zumindest eine seitliche Ansaugöffnung als luftdurchlässigen Bereich ausbildet. Die angesaugte Luft tritt somit durch die zumindest eine seitliche Ansaugöffnung aus einer im Wesentlichen senkrecht auf den lichten Querschnitt des geschlossenen Armbandes orientierten Richtung in die Kühlvorrichtung ein und wird in weiterer Folge in die Ausströmrichtung umgelenkt. Die hierfür vorgesehene Abdeckung unterstützt diese Umlenkung der angesaugten Luft und stellt sie auch dann sicher, wenn die erfindungsgemäße Kühlvorrichtung etwa unter einem langärmeligen Hemd oder einer Bluse getragen wird. Die Abdeckung schützt ferner den Luftstromgenerator vor einer die Luftstromerzeugung hemmenden Beeinflussung durch die über der Kühlvorrichtung getragene Bekleidung oder bei Aufliegen des Unterarmes auf einer Tischplatte oder dergleichen.

Der Luftstromerzeuger kann etwa als Piezo-Lüfter ausgeführt sein. Diese Kühler beruhen auf dem piezoelektrischen Effekt und sind auch als "Dual Piezoelectric Cooling Jets" (DCJ) bekannt. Sie weisen zwei dünne Metallplatten mit Piezoelementen auf, die sich beim Anlegen einer Wechselspannung mit Frequenzen von mehreren hundert Hertz bis Kilohertz bewegen und einen Hochgeschwindigkeits-Luftstrom erzeugen, der über eine Düse abgegeben wird. Ähnlich einem Blasebalg wird dabei Umgebungsluft eingesaugt und als Kühlluft wieder abgegeben. Vorzugsweise werden Piezo-Lüfter verwendet, die mit Frequenzen oberhalb von 20kHz betrieben werden, sodass sie für das menschliche Ohr nicht wahrnehmbar sind.

Bei Verwendung solcher Piezo-Lüfter wird vorgeschlagen, dass der zumindest eine Fortsatz düsenförmig ausgebildet ist. Der zumindest eine düsenförmige Fortsatz ist in Richtung der Hautoberfläche gerichtet, sodass die Hautoberfläche mit Kühlluft angeströmt oder umströmt wird. Hierbei kann der Düseneffekt ausgenutzt werden, bei dem stark bewegte Luft die umgebende Luft mitreißt und damit die Lüfterwirkung vervielfacht.

Der Luftstromerzeuger kann aber auch als Axialventilator ausgeführt sein, der in einem Durchbruch einer als Lagerplatte ausgeführten Halterung mit zur Lagerplatte senkrechter Drehachse gelagert ist. Die Abdeckung wird dabei bevorzugt als eine den Axialventilator überspannende Abdeckplatte ausgeführt. Diese Abdeckplatte begünstigt das ausschließliche Ansaugen von Luft durch die seitlichen Ansaugöffnungen und erhöht die Durchströmgeschwindigkeit der angesaugten Luft bei gleicher Drehzahl des Axialventilators. Die seitlichen Ansaugöffnungen können dabei auch durch die Ansaugöffnung überspannende Lamellen abgedeckt sein, die den Axialventilator vor dem Eindringen schädlicher Kleinteile schützen und bei geeigneter Ausführung auch Spritzwasser fernhalten können, aber die einströmende Luft ungehindert passieren lassen.

Die Fortsätze können auch als senkrecht zum lichten Querschnitt des geschlossenen Armbandes verlaufende Stege ausgeführt sein. Eine solche Ausführung begünstigt die Einstellung einer senkrecht zum lichten Querschnitt des geschlossenen Armbandes orientierten Ausströmrichtung der abzuführenden Luft. Dabei können die Stege auch gekrümmt ausgeführt sein, insbesondere in Form von Stegen, die in ihren mittleren Bereichen näher aneinander liegen als in ihren äußeren Endbereichen. Diese Ausführung begünstigt den Ausstoß der angewärmten Luft. Als besonders vorteilhaft hat es sich auch erwiesen, wenn die Fortsätze wärmeleitend ausgeführt sind, etwa aus metallischem Material, da sie auf diese Weise Wärme von der im Gebrauch anliegenden Hautoberfläche aufnehmen und auf den Luftstrom abgeben können.

Der Luftstromerzeuger kann durch ein entsprechendes Bedienelement oder durch bloßes Schließen des Armbandes in Betrieb gesetzt werden. Um jedoch einen Gewöhnungseffekt des Trägers und somit verringerte Kühlwirkung zu vermeiden wird vorgeschlagen, dass das Armband mit einer Steuereinheit versehen ist, die zur Verwirklichung von Ein- und Ausschaltzyklen mit dem Luftstromerzeuger verbunden ist. Bei jedem erneuten Einschalten des Luftstromerzeugers wird die Kühlung wieder subjektiv intensiv wahrgenommen. Ein Luftstromerzeuger hat dabei gegenüber einem Peltier-Element den Vorteil, dass der Kühleffekt ohne Verzögerung unmittelbar nach dem Ein- oder Ausschalten aktiviert oder deaktiviert werden kann, sodass eine Steuerung gut möglich ist. Zudem verringert sich mithilfe von Ein- und Ausschaltzyklen der Strombedarf für den Luftstromerzeuger und die Betriebsdauer kann deutlich verlängert werden. Des Weiteren kann das Armband auch mit Puls- und/oder Temperatursensoren versehen sein, die mit der Steuereinheit verbunden sind. Diese Sensoren messen die Haut- und/oder die Umgebungstemperaturen und können anhand der Messwerte mithilfe der Steuereinheit Kühlzeiten, Kühldauer und Kühlphasen so einstellen, dass ein optimaler subjektiver Kühleffekt erzielt wird. Auch Feuchtigkeitsmesser, Lagesensoren und ähnliches können vorgesehen sein und deren Daten zur Optimierung des Kühleffektes herangezogen werden. Diese Sensoren können in der Halterung eingebaut werden, in den Fortsätzen der Halterung oder in anderen Teilen des Armbandes.

Freilich kann das erfindungsgemäß vorgesehene Armband auch als Träger anderer Vorrichtungen dienen, etwa für Uhren mit Zusatzfunktionen ("Smartwatches"), Solarzellen oder tragbarer Kommunikationsgeräte. Die für den Luftstromerzeuger vorgesehene Stromversorgung kann dabei auch als Stromversorgung der zusätzlichen Vorrichtung dienen, oder die für die zusätzliche Vorrichtung vorgesehene Stromversorgung als Stromversorgung für den Luftstromerzeuger.

Die Erfindung wird in weiterer Folge anhand von Ausführungsbeispielen mithilfe der beiliegenden Figuren näher erläutert. Es zeigen dabei die
Fig. 1 eine perspektivische Ansicht einer Ausführungsform der erfindungsgemäßen Kühlvorrichtung mit Axialventilator,
Fig. 2 eine perspektivische Ansicht einer Ausführungsform der erfindungsgemäßen Kühlvorrichtung während des Gebrauches,
Fig. 3 eine Schnittansicht durch die Ausführungsform des Kühlers gemäß Fig. 1,
Fig. 4 eine perspektivische Ansicht einer weiteren Ausführungsform der erfindungsgemäßen Kühlvorrichtung,
Fig. 5 eine Schnittansicht einer Ausführungsform der erfindungsgemäßen Kühlvorrichtung in Gebrauchslage zur Erläuterung des generierten Luftstromes,
Fig. 6a und 6b perspektivische Ansichten der erfindungsgemäßen Kühlvorrichtung mit unterschiedlichen Ausführungen der Abstandshalter,
Fig. 7 eine Schnittansicht durch eine Ausführungsform des Kühlers,
Fig. 8a und 8b Darstellungen zur Erläuterung möglicher Ausführungsformen von Dämpfungselementen,
Fig. 9 eine perspektivische Ansicht einer weiteren Ausführungsform der erfindungsgemäßen Kühlvorrichtung mit einer Smartwatch,
Fig. 10 eine perspektivische Ansicht einer weiteren Ausführungsform der erfindungsgemäßen Kühlvorrichtung mit zusätzlichen Sensoren,
Fig. 11 eine Darstellung des Kühlers zur Erläuterung einer möglichen Ausführung der Ansaugöffnungen,
Fig. 12 eine Schnittansicht durch eine weitere Ausführungsform einer erfindungsgemäßen Kühlvorrichtung mit einem Piezo-Lüfter,
Fig. 13 eine Schnittansicht einer erfindungsgemäßen Ausführungsform mit Piezo-Lüfter und einer möglichen Ausführungsform eines düsenförmig ausgeführten Fortsatzes,
Fig. 14 eine Schnittansicht einer weiteren Ausführungsform eines düsenförmig ausgeführten Fortsatzes bei einem Piezo-Lüfter,
Fig. 15 eine Schnittansicht einer erfindungsgemäßen Ausführungsform mit Piezo-Lüfter und einer weiteren Ausführungsform der düsenförmig ausgeführten Fortsätze,
Fig. 16 eine Schnittansicht in Richtung A-A der Fig. 15,
Fig. 17 eine Schnittansicht einer erfindungsgemäßen Ausführungsform mit Piezo-Lüfter und einer weiteren Ausführungsform eines düsenförmig ausgeführten Fortsatzes mit nach unten orientierter Ansaugöffnung, und die
Fig. 18 eine Schnittansicht einer erfindungsgemäßen Ausführungsform mit Piezo-Lüfter und einer weiteren Ausführungsform düsenförmig ausgeführter Fortsätze und nach unten orientierter Ansaugöffnungen.

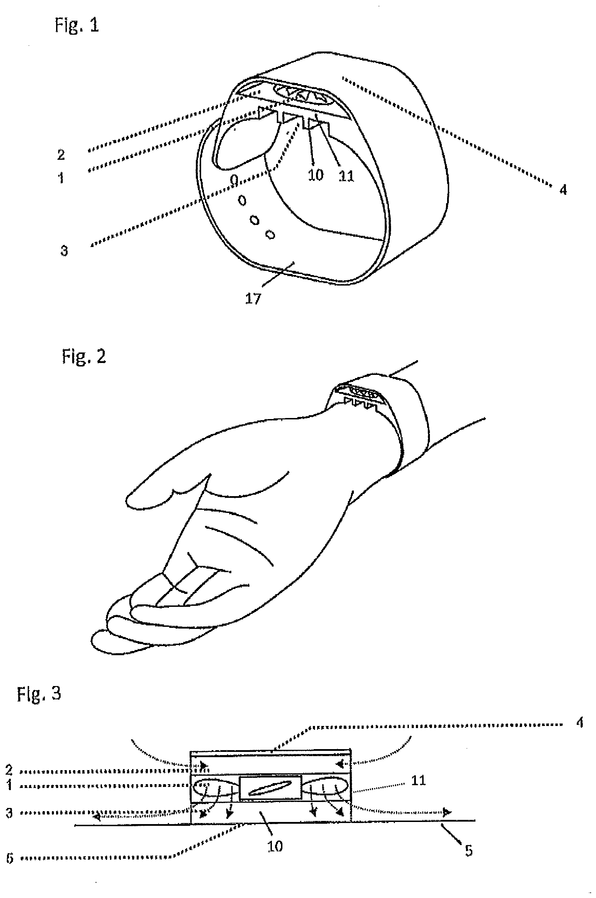

Zunächst wird auf die Fig. 1 Bezug genommen, die eine perspektivische Ansicht einer Ausführungsform der erfindungsgemäßen Kühlvorrichtung mit einem Armband 17 und einer am Armband 17 befestigten oder angeformten Halterung 11 zeigt, die im gezeigten Ausführungsbeispiel als Lagerplatte ausgeführt ist. Die Halterung 11 bildet dabei bei geschlossenem Armband 17 einen Teil des Umfangsbereiches des Armbandes 17. In der als Lagerplatte ausgeführten Halterung 11 ist ein als Axialventilator ausgeführter Luftstromerzeuger 1 drehbar gelagert, wobei die Drehachse des Axialventilators und somit die Ausströmrichtung des erzeugten Luftstromes senkrecht zur Lagerplatte orientiert ist.

Die Halterung 11 ist auf ihrer bei geschlossenem Armband 17 nach außen orientierten Seite mit einer Abdeckung 4 versehen, die mit der Halterung 11 zumindest eine seitliche Ansaugöffnung 2 ausbildet. Die Halterung 11 und die Abdeckung 4 sind hierfür aus einem hinreichend steifen Material gefertigt. Die Abdeckung 4 ist im gezeigten Ausführungsbeispiel als eine den Axialventilator überspannende Abdeckplatte ausgeführt.

Auf der bei geschlossenem Armband 17 nach innen orientierten Seite der Halterung 11 sind in Ausströmrichtung abstehende Fortsätze 10 angeordnet. Im gezeigten Ausführungsbeispiel sind die Fortsätze 10 als zwei senkrecht zum lichten Querschnitt des geschlossenen Armbandes 17 verlaufende Stege ausgeführt, die an der Halterung 11 angeformt sind. Bei angelegtem Armband 17 dienen die Fortsätze 10 auch als Abstandshalter, um eine ausreichende Beabstandung der Halterung 11 von der Hautoberfläche sicher zu stellen und unterhalb der Halterung 11 einen Kühlraum für den erzeugten Luftstrom zu bilden, wie anhand der Fig. 2 ersichtlich ist.

Die Fig. 2 zeigt eine perspektivische Ansicht einer Ausführungsform der erfindungsgemäßen Kühlvorrichtung während des Gebrauches, wobei ersichtlich ist, dass das Armband 17 in üblicher Weise im Handgelenksbereich angelegt wird, wobei sich der Luftstromerzeuger 1 über dem Pulspunkt des Handgelenks befindet, also jenem Bereich, in dem der Puls gefühlt werden kann. Wie bereits erwähnt wurde, hat die Kühlung von Pulspunkten einen großen Einfluss auf das subjektive Wärmeempfinden des Menschen. Durch die angeströmte Luft fühlt sich der Träger der erfindungsgemäßen Kühlvorrichtung subjektiv kühler, ohne dass der gesamte Körper gekühlt werden muss. Einer der empfindlichsten Pulspunkte liegt dabei an der Handwurzel, was durch die erfindungsgemäße Kühlvorrichtung ausgenutzt wird. Die Fortsätze 10 liegen während des Gebrauches der erfindungsgemäßen Kühlvorrichtung dicht am Handgelenk an und stellen die Beabstandung der Halterung 11 mit dem Luftstromerzeuger 1 von der Hautoberfläche 5 sicher (siehe auch Fig. 3). Vorzugsweise können die Fortsätze 10 wärmeleitend ausgeführt sein, sodass sie Wärme von der Hautoberfläche 5 abführen und an den vorbeiströmenden Luftstrom abgeben können.

Anhand der Fig. 3, die eine Schnittansicht durch die Ausführungsform des Kühlers gemäß Fig. 1 zeigt, wird der sich einstellende Luftstrom durch den Kühler erläutert. Der Axialventilator fördert den Luftstrom von der Ansaugseite zur Druckseite parallel zur Drehachse des Axialventilators. Aufgrund der von der Halterung 11 beabstandeten Abdeckung 4, die zwischen der Abdeckung 4 und der Halterung 11 zumindest eine seitliche Ansaugöffnung 2 ausbildet, tritt die angesaugte Luft jedoch durch die zumindest eine seitliche Ansaugöffnung 2 senkrecht zum lichten Querschnitt des geschlossenen Armbandes 17 in die Kühlvorrichtung ein und wird in weiterer Folge durch den Axialventilator in die Drehachsrichtung umgelenkt und in die hierzu parallele Ausströmrichtung auf die Druckseite ausgeworfen. Auf der bei geschlossenem Armband 17 nach innen orientierten Seite der Halterung 11 wird der so erzeugte Luftstrom durch die erfindungsgemäß in Ausströmrrichtung von der Halterung 11 abstehenden Fortsätze 10 sichergestellt. Auf diese Weise wird bei angelegtem Armband 17 ein Kühlbereich für den Luftstrom zwischen der Halterung 11 und der Hautoberfläche 5 geschaffen, wobei durch das senkrechte Anströmen der Hautoberfläche 5 mit dem kühlenden Luftstrom ein maximaler Kühleffekt erreicht wird und aufgrund der Beabstandung der Halterung 11 von der Hautoberfläche 5 seitliche Abgabeöffnungen 3 bereit gestellt werden, durch die die angewärmte Luft die erfindungsgemäße Kühlvorrichtung verlassen kann.

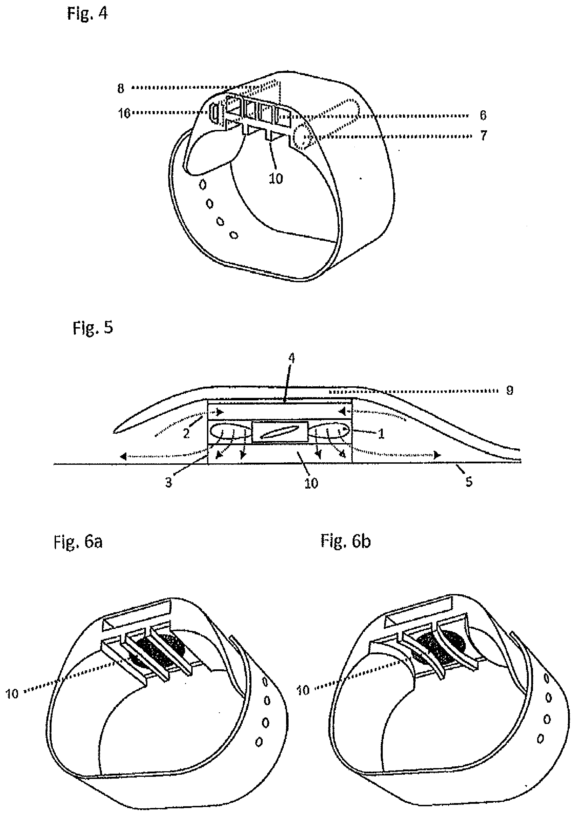

Die Fig. 4 zeigt eine perspektivische Ansicht einer weiteren Ausführungsform der erfindungsgemäßen Kühlvorrichtung, wobei die seitliche Ansaugöffnung 2 mit die Ansaugöffnung 2 überspannende Lamellen 6 versehen ist. Des Weiteren sind eine im Armband 17 angeordnete Steuereinheit 7 sowie eine Stromversorgung 8 mit einer Ladebuchse 16 ersichtlich. Die Stromversorgung 8 ist etwa als ein über einen Micro-USB aufladbarer Akkumulator ausgeführt. Die Steuereinheit 7, der Luftstromerzeuger 1 sowie die Stromversorgung 8 sind über nicht dargestellte elektrische Leiter miteinander verbunden. Die Steuereinheit 7 kann etwa so ausgelegt sein, dass sie Ein- und Ausschaltzyklen des Luftstromerzeugers 1 steuert und somit einen Gewöhnungseffekt des Trägers und die damit verbundene Verringerung der subjektiven Kühlwirkung vermeidet. Zudem verringert sich der Strombedarf für den Luftstromerzeuger 1 und die Betriebsdauer kann deutlich verlängert werden. Die Steuereinheit 7 kann auch weitere Funktionen übernehmen, wie noch näher ausgeführt werden wird.

Anhand der Fig. 5 wird der generierte Luftstrom innerhalb einer Ausführungsform der erfindungsgemäßen Kühlvorrichtung mit Axialventilator in Gebrauchslage erläutert, wobei die Kühlvorrichtung unterhalb eines Hemdärmels 9 getragen wird. Es ist ersichtlich, wie die Abdeckung 4 den Axialventilator vor einer die Drehbewegung des Axialventilators hemmenden Beeinflussung durch die über der Kühlvorrichtung getragenen Bekleidung schützt. Die Umlenkung der angesaugten Luft von einer senkrecht zum lichten Querschnitt des geschlossenen Armbandes 17 gerichteten Ansaugrichtung in einen senkrecht zur Hautoberfläche 5 orientierten Kühlluftstrom wird durch das Zusammenwirken der Abdeckung 4, dem Axialventilator und den Fortsätzen 10 bewirkt.

In der Fig. 6a und 6b sind perspektivische Ansichten der erfindungsgemäßen Kühlvorrichtung mit unterschiedlichen Ausführungen der Fortsätze 10 dargestellt. Die Fortsätze 10 können wie bereits erwähnt als senkrecht zum lichten Querschnitt des geschlossenen Armbandes 17 verlaufende Stege ausgeführt sein, wobei in der Fig. 6a beispielsweise zwei solcher Stege dargestellt sind. Eine solche Ausführung begünstigt die Einstellung einer senkrecht zum lichten Querschnitt des geschlossenen Armbandes 17 orientierten Abgaberichtung der abzuführenden Luft. Dabei können die Stege auch gekrümmt ausgeführt sein, wie in der Fig. 6b ersichtlich ist. Die Krümmung ist dabei so gewählt, dass die Stege in ihren mittleren Bereichen näher aneinander liegen als in ihren äußeren Endbereichen, sodass sich der lichte Querschnitt der Abgabeöffnungen 3 von den mittleren Bereichen zu den Endbereichen der Stege erweitert. Der erweiterte Querschnitt der Abgabeöffnungen 3 begünstigt den Ausstoß der ausströmenden Luft, da die Strömungsgeschwindigkeit der Luft in Richtung der Abgabeöffnungen 3 abnimmt und das sich erweiternde Volumen Rückstaueffekte verringert. Die als Stege ausgeführten Fortsätze 10 verlaufen dabei zumindest abschnittsweise innerhalb des Luftstroms.

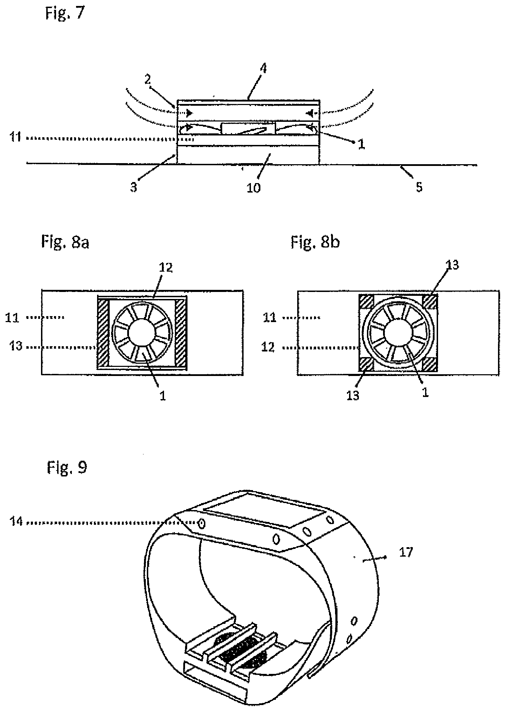

Die Fig. 7 zeigt eine Schnittansicht durch eine Ausführungsform eines als Axialventilator ausgeführten Luftstromerzeugers, wobei ersichtlich ist, dass der Axialventilator zumindest entlang seines der zumindest einen Ansaugöffnung 2 zugewandten Umfangsbereiches die als Lagerplatte ausgeführte Halterung 11 auf ihrer Ansaugseite überragt. Diese Maßnahme erhöht das Ansaugvolumen für die angesaugte Luft und somit die Kühlluftmenge bei gleicher Drehzahl des Axialventilators.

Hinsichtlich des Axialventilators sind Ausführungen verfügbar, die sich durch hohe Laufruhe und leisen Betrieb auszeichnen. Um dennoch allfällige Vibrationen des Axialventilators aufzunehmen und ihre Übertragung auf das Handgelenk des Trägers zu vermeiden können elastische Dämpfungselemente 13 vorgesehen sein, die in den Kontaktbereichen zwischen einem den Axialventilator aufnehmenden Gehäuse 12 und der Halterung 11 angeordnet sind. Die Fig. 8a und 8b zeigen mögliche Ausführungsformen dieser Dämpfungselemente 13, die etwa durch dünne Gummilamellen gebildet werden können und beispielsweise als randseitige Streifenlamellen (Fig. 8a) oder als Ecklamellen (Fig. 8b) ausgeführt sein können. Diese Dämpfungselemente 13 nehmen ferner Stoßkräfte auf, die durch das Tragen an der Hand entstehen. Auf diese Weise wird das Lager des Axialventilators geschont.

Wie bereits erwähnt wurde kann das erfindungsgemäß vorgesehene Armband 17 auch als Träger anderer Geräte 14 dienen, etwa für Uhren mit Zusatzfunktionen ("Smartwatches") oder für tragbare Kommunikationsgeräte. Die Fig. 9 zeigt etwa die Verwendung einer erfindungsgemäßen Kühlvorrichtung mit einer Smartwatch. Die erfindungsgemäße Kühlvorrichtung und das zusätzliche Gerät 14 befinden sich dabei an gegenüberliegenden Seiten des Armbandes 17. Es ist dabei möglich, dieselben Komponenten für die Kühlvorrichtung und das zusätzliche Gerät 14 zu verwenden. So kann etwa die für den Luftstromerzeuger 1 vorgesehene Stromversorgung 8 auch als Stromversorgung des zusätzlichen Geräts 14 dienen, oder die für das zusätzliche Gerät 14 vorgesehene Stromversorgung als Stromversorgung 8 für den Luftstromerzeuger 1.

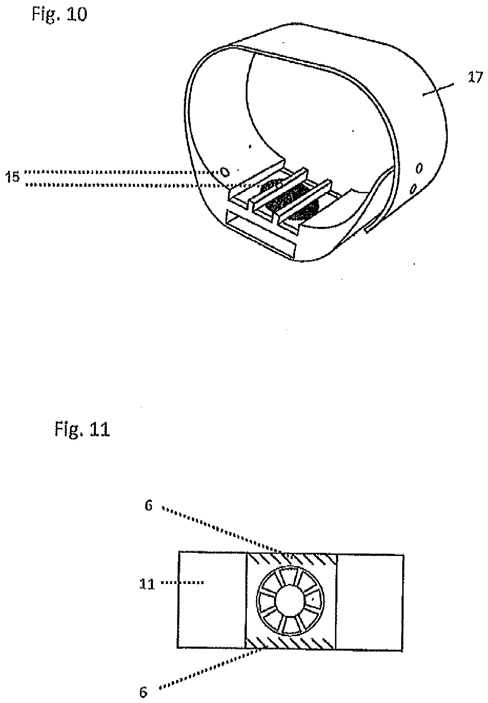

Wie anhand der Fig. 10 ersichtlich ist, kann die erfindungsgemäße Kühlvorrichtung auch mit Puls- und/oder Temperatursensoren 15 versehen sein, die mit der Steuereinheit 7 verbunden sind. Diese Puls- und/oder Temperatursensoren 15 messen die Haut- und/oder die Umgebungstemperaturen und können anhand der Messwerte mithilfe der Steuereinheit 7 Kühlzeiten, Kühldauer und Kühlphasen so einstellen, dass ein optimaler subjektiver Kühleffekt erzielt wird. Auch Feuchtigkeitsmesser, Lagesensoren und ähnliches können vorgesehen sein und deren Daten zur Optimierung des subjektiven Kühleffektes herangezogen werden. Diese Sensoren sowie die genannten Puls- und/oder Temperatursensoren 15 können in den Fortsätzen 10 eingebaut werden, in der Halterung 11 oder in anderen Teilen des Armbandes 17.

In der Fig. 11 ist eine weitere Ausführung der die seitliche Ansaugöffnung 2 überspannenden Lamellen 6 dargestellt. Die in Form einer Schar zueinander parallel angeordneter Lamellen 6 schützen den als Axialventilator ausgeführten Luftstromerzeuger 1 vor dem Eindringen schädlicher Kleinteile und bei geeigneter Ausführung auch vor Spritzwasser, lassen aber die einströmende Luft ungehindert passieren.

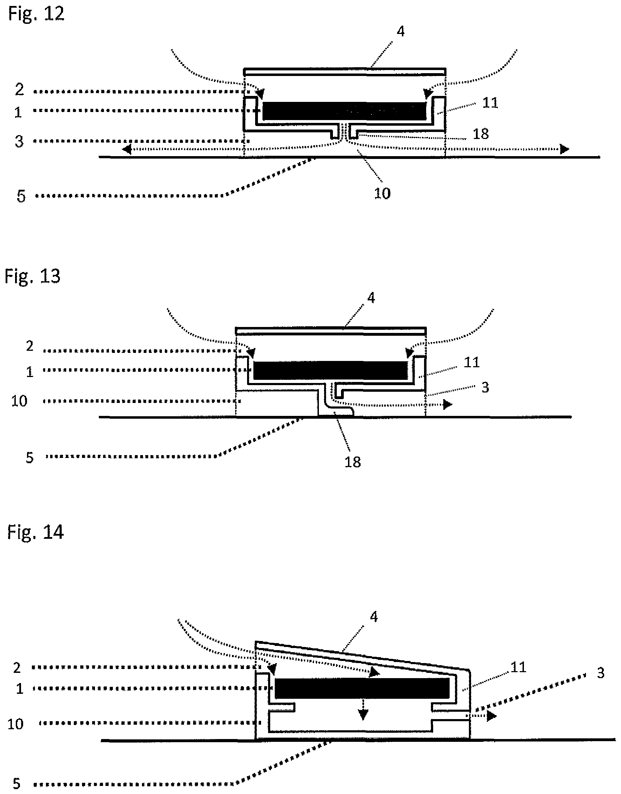

Eine weitere Ausführungsform einer erfindungsgemäßen Kühlvorrichtung mit einem als Piezo-Lüfter ausgeführten Luftstromerzeuger 1 wird anhand der Fig. 12 bis 18 erläutert. Der Piezo-Lüfter ist in einer Halterung 11 angeordnet, die auf ihrer bei geschlossenem Armband 17 nach außen orientierten Seite mit einer Abdeckung 4 versehen ist, die mit der Halterung 11 zumindest eine seitliche Ansaugöffnung 2 ausbildet. Die Halterung 11 kann ferner mit einer düsenförmig ausgebildeten Ausströmöffnung 18 versehen sein, durch die der vom Luftstromerzeuger 1 erzeugte Luftstrom austritt. Auf der bei geschlossenem Armband 17 nach innen orientierten Seite der Halterung 11 sind nach innen abstehende Fortsätze 10 angeordnet, die bei angelegtem Armband 17 wiederum als Abstandshalter zur Hautoberfläche 5 dienen. In der Regel wird bei einer solchen Ausführungsform die Ausströmöffnung 18 zwischen zwei der Fortsätze 10 angeordnet sein, sodass die Fortsätze 10 den Luftstrom zumindest abschnittweise umschließen.

Wie in der Fig. 13 gezeigt ist, kann die düsenförmig ausgeführte Ausströmöffnung 18 auch verlängert ausgeführt sein, sodass sie selbst auch als Abstandshalter fungiert, wobei der abgegebene Luftstrom so durch die düsenförmig ausgeführte Ausströmöffnung 18 umgelenkt wird, dass der erzeugte Luftstrom parallel zur Hautoberfläche 5 aus der Abgabeöffnung 3 austritt und die Hautoberfläche 5 kühlend umspült.

Die Fig. 14 zeigt eine Schnittansicht einer weiteren Ausführungsform eines Fortsatzes 10 mit einer düsenförmig ausgeführten Abgabeöffnung 3, die so ausgeführt ist, dass der erzeugte Luftstrom wiederum parallel zur Hautoberfläche 5 aus der Abgabeöffnung 3 austritt und die Hautoberfläche 5 kühlend umspült. In dieser Ausführungsform umschließt der Fortsatz 10 den erzeugten Luftstrom zur Gänze.

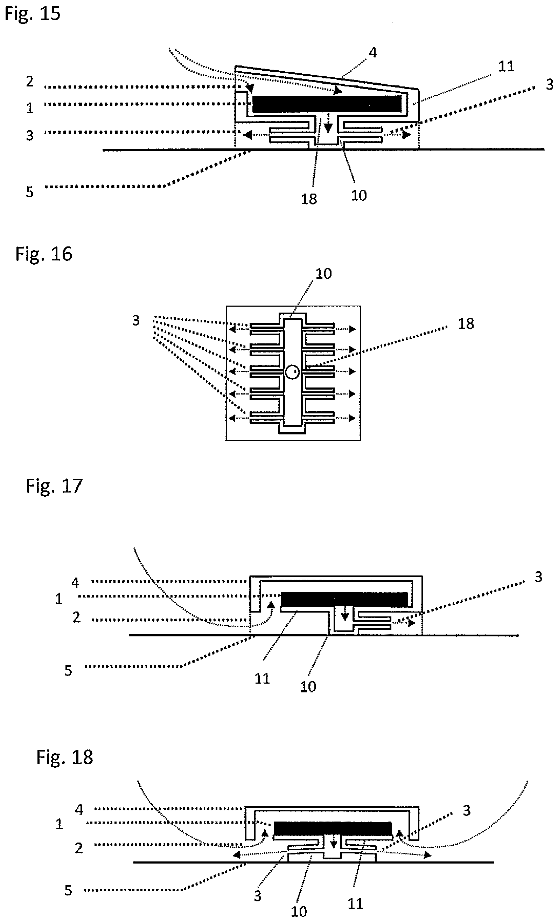

Die Fig. 15 und 16 zeigen Schnittansichten einer erfindungsgemäßen Ausführungsform mit einem als Piezo-Lüfter ausgeführten Luftstromerzeuger 1 und einer weiteren Ausführungsform der Fortsätze 10, die im gezeigten Ausführungsbeispiel über mehrere düsenförmig ausgebildete Abgabeöffnungen 3 verfügen, wobei der erzeugte Luftstrom wiederum parallel zur Hautoberfläche 5 aus den Abgabeöffnungen 3 austritt und die Hautoberfläche 5 kühlend umspült. Auch in dieser Ausführungsform umschließt der Fortsatz 10 den erzeugten Luftstrom zur Gänze und bildet einen Abgaberaum, bis er an den Abgabeöffnungen 3 austritt.

Die Fig. 17 zeigt eine Schnittansicht einer erfindungsgemäßen Ausführungsform mit einem als Piezo-Lüfter ausgeführten Luftstromerzeuger 1 und einer weiteren Ausführungsform eines Fortsatzes 10 mit einer düsenförmig ausgeführten Abgabeöffnung 3, wobei die Abgabeöffnung 3 wiederum so ausgeführt ist, dass der erzeugte Luftstrom parallel zur Hautoberfläche 5 aus der Abgabeöffnung 3 austritt und die Hautoberfläche 5 kühlend umspült. Des Weiteren ist eine weitere Ausführungsform der Ansaugöffnung 2 ersichtlich, die sich nun auf der bei geschlossenem Armband 17 nach innen orientierten Seite der Halterung 11 befindet. Bei geschlossenem Armband 17 wird dabei Luft entlang der Hautoberfläche 5 angesaugt, die durch die Ansaugöffnung 2 in die Kühlvorrichtung eintritt. In weiterer Folge wird sie durch den als Piezo-Lüfter ausgeführten Luftstromerzeuger 1 stoßartig beschleunigt und tritt durch den düsenförmig ausgeführten Fortsatz 10 wieder aus der Kühlvorrichtung aus. Der düsenförmig ausgeführte Fortsatz 10 dient dabei auch als Abstandshalter, um eine ausreichende Beabstandung der Halterung 11 von der Hautoberfläche 5 sicher zu stellen und die Ansaugöffnung 2 somit freizuhalten.

Die Fig. 18 zeigt eine zu Fig. 17 vergleichbare, aber symmetrisch ausgeführte Ausführungsform. Wiederum wird bei geschlossenem Armband 17 Luft entlang der Hautoberfläche 5 angesaugt, die in weiterer Folge durch die Ansaugöffnung 2 in die Kühlvorrichtung eintritt. In weiterer Folge wird sie durch den als Piezo-Lüfter ausgeführten Luftstromerzeuger 1 stoßartig beschleunigt und tritt durch den düsenförmig ausgeführten Fortsatz 10 wieder aus der Kühlvorrichtung aus. Der düsenförmig ausgeführte Fortsatz 10 ist nun aber symmetrisch ausgeführt und gibt den kühlenden Luftstrom beidseits der Kühlvorrichtung in entgegen gesetzte Richtungen ab. Der Fortsatz 10 dient dabei auch als Abstandshalter, um eine ausreichende Beabstandung der Halterung 11 von der Hautoberfläche 5 sicher zu stellen und die Ansaugöffnung 2 somit freizuhalten.

Die Erfindung stellt somit eine tragbare Kühlvorrichtung bereit, die überaus klein und kompakt ausgeführt werden kann und somit unauffällig etwa auch im beruflichen Bereich unter entsprechender Bekleidung getragen werden kann. Die erfindungsgemäße Vorrichtung ist ferner robust und widerstandsfähig und somit alltagstauglich und weist dabei einen im Vergleich zu herkömmlichen Geräten geringeren Stromverbrauch auf und verfügt somit über längere Betriebszeiten.

### Bezugszeichenliste:

- 1: Luftstromerzeuger
- 2: Ansaugöffnung
- 3: Abgabeöffnung
- 4: Abdeckung
- 5: Hautoberfläche
- 6: Lamellen
- 7: Steuereinheit
- 8: Stromversorgung
- 9: Hemdärmel
- 10: Fortsätze
- 11: Halterung
- 12: Gehäuse
- 13: Dämpfungselemente
- 14: Zusätzliches Gerät
- 15: Puls- und/oder Temperatursensoren
- 16: Ladebuchse
- 17: Armband
- 18: Ausströmöffnung

## Patentansprüche

1. Tragbare Kühlvorrichtung mit einem Armband (17) und einem am Armband (17) angeordneten Kühler, **dadurch gekennzeichnet, dass** der Kühler als Luftstromerzeuger (1) für einen in eine Ausströmrichtung des Kühlers gerichteten Luftstrom ausgeführt ist, wobei der Luftstromerzeuger (1) in einer am Armband (17) befestigten oder angeformten Halterung (11) angeordnet ist, die zumindest einen in Ausströmrichtung abstehenden Fortsatz (10) aufweist, der zumindest abschnittsweise im oder am Luftstrom oder den Luftstrom zumindest abschnittsweise umschließend verläuft.

2. Tragbare Kühlvorrichtung nach Anspruch 1, **dadurch gekennzeichnet, dass** die Halterung (11) auf ihrer bei geschlossenem Armband (17) nach außen orientierten Seite mit einer Abdeckung (4) versehen ist, die luftdurchlässige Bereiche aufweist.

3. Tragbare Kühlvorrichtung nach Anspruch 1 oder 2, **dadurch gekennzeichnet, dass** der Luftstromerzeuger (1) als Piezo-Lüfter ausgeführt ist.

4. Tragbare Kühlvorrichtung nach einem der Ansprüche 1 bis 3, **dadurch gekennzeichnet, dass** der zumindest eine Fortsatz (10) düsenförmig ausgebildet ist.

5. Tragbare Kühlvorrichtung nach Anspruch 1 oder 2, **dadurch gekennzeichnet, dass** der Luftstromerzeuger (1) als Axialventilator ausgeführt ist, der in einem Durchbruch einer als Lagerplatte ausgeführten Halterung (11) mit zur Lagerplatte senkrechter Drehachse gelagert ist.

6. Tragbare Kühlvorrichtung nach einem der Ansprüche 1 bis 5, **dadurch gekennzeichnet, dass** mehrere Fortsätze (10) vorgesehen sind, die als senkrecht zum lichten Querschnitt des geschlossenen Armbandes (17) verlaufende Stege ausgeführt sind.

7. Tragbare Kühlvorrichtung nach Anspruch 6, **dadurch gekennzeichnet, dass** die Stege gekrümmt ausgeführt sind.

8. Tragbare Kühlvorrichtung nach einem der Ansprüche 1 bis 7, **dadurch gekennzeichnet, dass** der zumindest eine Fortsatz (10) wärmeleitend ausgeführt ist.

9. Tragbare Kühlvorrichtung nach einem der Ansprüche 1 bis 8, **dadurch gekennzeichnet, dass** das Armband (17) mit einer Steuereinheit (7) versehen ist, die zur Verwirklichung von Ein- und Ausschaltzyklen mit dem Luftstromerzeuger (1) verbunden ist.

10. Tragbare Kühlvorrichtung nach Anspruch 9, **dadurch gekennzeichnet, dass** das Armband (17) mit Puls- und/oder Temperatursensoren (15) versehen ist, die mit der Steuereinheit (7) verbunden sind.

## Claims

1. Wearable cooling device, having a wristband (17) and a cooler arranged on the wristband (17), **characterized in that** the cooler is designed as an airflow generator (1) for an airflow directed in an outflow direction of the cooler, wherein the airflow generator (1) is arranged in a holder (11) fastened to or integrally formed on the wristband (17), said holder having at least one extension (10) which projects in the outflow direction and extends at least in sections in or on the airflow or by at least partially enclosing the airflow.

2. Wearable cooling device according to claim 1, **characterized in that** the holder (11) is provided with a cover (4) on its side oriented outwardly when the wristband (17) is closed, said cover having air-permeable areas.

3. Wearable cooling device according to claim 1 or 2, **characterized in that** the airflow generator (1) is designed as a piezo fan.

4. Wearable cooling device according to one of the claims 1 to 3, **characterized in that** the at least one extension (10) is nozzle-shaped.

5. Wearable cooling device according to claim 1 or 2, **characterized in that** the airflow generator (1) is designed as an axial fan, which is mounted in a breakthrough of a holder (11) formed as a bearing plate and is mounted with a vertical axis of rotation in relation to the bearing plate.

6. Wearable cooling device according to one of the claims 1 to 5, **characterized in that** a plurality of extensions (10) are provided, which are designed as webs extending perpendicularly to the clear cross-section of the closed wristband (17).

7. Wearable cooling device according to claim 6, **characterized in that** the webs are curved.

8. Wearable cooling device according to one of the claims 1 to 7, **characterized in that** the at least one extension (10) is designed in a thermally conductive manner.

9. Wearable cooling device according to one of the claims 1 to 8, **characterized in that** the wristband (17) is provided with a control unit (7) which is connected to the airflow generator (1) for realizing switching-on and switching-off cycles.

10. Wearable cooling device according to claim 9, **characterized in that** the wristband (17) is provided with pulse and/or temperature sensors (15), which are connected to the control unit (7).

## Revendications

1. Dispositif de refroidissement portable comportant un bracelet (17) et un refroidisseur disposé sur le bracelet (17), **caractérisé en ce que** le refroidisseur est réalisé sous la forme d'un générateur de flux d'air (1) pour un flux d'air dirigé dans une direction de sortie du refroidisseur, le générateur de flux d'air (1) étant disposé dans un support (11) fixé ou formé sur le bracelet (17), qui comporte au moins une extension (10) qui fait saillie dans la direction de sortie et s'étend au moins sur certaines parties dans ou le long du flux d'air ou s'étend au moins sur certaines parties de façon à entourer le flux d'air.

2. Dispositif de refroidissement portable selon la revendication 1, **caractérisé en ce que** le support (11) est pourvu, sur son côté orienté vers l'extérieur lorsque le bracelet (17) est fermé, d'un couvercle (4) qui comporte des zones perméables à l'air.

3. Dispositif de refroidissement portable selon la revendication 1 ou 2, **caractérisé en ce que** le générateur de flux d'air (1) est réalisé sous la forme d'un ventilateur piézoélectrique.

4. Dispositif de refroidissement portable selon l'une des revendications 1 à 3, **caractérisé en ce que** ladite au moins une extension (10) est réalisée en forme de buse.

5. Dispositif de refroidissement portable selon la revendication 1 ou 2, **caractérisé en ce que** le générateur de flux d'air (1) est réalisé sous la forme d'un ventilateur axial qui est monté dans une ouverture d'un support (11) réalisé sous la forme d'une plaque d'appui et ayant un axe de rotation perpendiculaire à la plaque d'appui.

6. Dispositif de refroidissement portable selon l'une des revendications 1 à 5, **caractérisé en ce qu'**il est prévu plusieurs extensions (10) qui sont réalisées sous la forme de nervures s'étendant perpendiculairement à la section transversale libre du bracelet (17) fermé.

7. Dispositif de refroidissement portable selon la revendication 6, **caractérisé en ce que** les nervures sont courbes.

8. Dispositif de refroidissement portable selon l'une des revendications 1 à 7, **caractérisé en ce que** ladite au moins une extension (10) est conductrice de chaleur.

9. Dispositif de refroidissement portable selon l'une des revendications 1 à 8, **caractérisé en ce que** le bracelet (17) est pourvu d'une unité de commande (7) qui est reliée au générateur de flux d'air (1) pour réaliser des cycles de mise en marche et d'arrêt.

10. Dispositif de refroidissement portable selon la revendication 9, **caractérisé en ce que** le bracelet (17) est pourvu de capteurs de pouls et/ou de température (15) qui sont reliés à l'unité de commande (7).
